# EUROPÄISCHE PATENTSCHRIFT

(11) **EP 0 754 680 B1**
(45) Veröffentlichungstag und Bekanntmachung des Hinweises auf die Patenterteilung: **02.01.2002**
(21) Anmeldenummer: 96111359.4
(22) Anmeldetag: 15.07.1996
(51) Int. Cl.: C07C 317/44, A61K 31/155

(54) **4-Fluoralkyl-substituierte Benzoylguandine, Verfahren zu ihrer Herstellung, ihre Verwendung als Medikament sowie sie enthaltendes Medikament**
4-Fluoroalkyl-substituted benzoylguanidines, process for their preparation, their use as medicament as well as medicaments containing them
Benzoylguanidines substituées en position 4 avec un groupement fluoroalkyle, leur utilisation comme médicament ainsi que médicaments les contenant

(30) Priorität: 19.07.1995 DE 19526381
(43) Veröffentlichungstag der Anmeldung: 22.01.1997
(73) Patentinhaber: HOECHST AKTIENGESELLSCHAFT, 65929 Frankfurt am Main (DE)
(72) Erfinder: Weichert, Andreas, Dr., 63329 Egelsbach (DE); Brendel, Joachim, Dr., 61118 Bad Vilbel (DE); Kleemann, Heinz-Werner, Dr., 65474 Bischofsheim (DE); Lang, Hans Jochen, Dr., 65719 Hofheim (DE); Schwark, Jan-Robert, Dr., 65929 Frankfurt (DE); Scholz, Wolfgang, Dr., 65760 Eschborn (DE); Albus, Udo, Dr., 61197 Florstadt (DE)

(56) Entgegenhaltungen:
- EP-A- 0 556 674
- EP-A- 0 612 723
- EP-A- 0 640 588

## Beschreibung

Die Erfindung betrifft Benzoylguanidine der Formel I 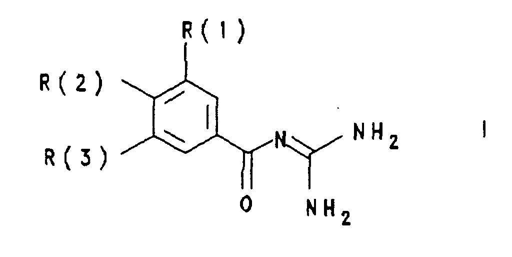 worin bedeuten:
- R(1): R(4)-SOₘ oder R(5)R(6)N-SO₂-;
m 1 oder 2;
R(4) und R(5) unabhängig voneinander Alkyl mit 1, 2, 3, 4, 5, 6, 7 oder 8 C-Atomen, Alkenyl mit 3, 4, 5 oder 6 C-Atomen, CF₃ oder -CₙH₂ₙ-R(7);
n Null, 1, 2, 3 oder 4;
R(6) H oder Alkyl mit 1, 2, 3 oder 4 C-Atomen;
R(7) Cycloalkyl mit 3, 4, 5, 6, 7 oder 8 C-Atomen oder Phenyl, welches nicht substituiert ist oder substituiert mit 1 - 3 Substituenten ausgewählt aus der Gruppe bestehend aus F, Cl, CF₃, Methyl, Methoxy und NR(8)R(9);
R(8) und R(9)
H oder Alkyl mit 1, 2, 3 oder 4 C-Atomen; oder
R(5) auch Wasserstoff;
oder
R(5) und R(6)
gemeinsam 4 oder 5 Methylengruppen, von denen eine CH₂-Gruppe durch Sauerstoff, S, NH, N-CH₃ oder N-Benzyl ersetzt sein kann; oder
- R(1): -SR(10);
R(10)
unabhängig voneinander Wasserstoff, Alkyl mit 1, 2, 3, 4, 5, 6, 7 oder 8 C-Atomen, -CₛH₂ₛ-(C₃-C₈)-Cycloalkyl oder ein aromatisches System ausgewählt aus der Gruppe bestehend aus Pyridyl, Pyrrolyl, Chinolyl, Isochinolyl, Imidazolyl oder Phenyl;
s Null, 1 oder 2; wobei die aromatischen Systeme Pyridyl, Pyrrolyl, Chinolyl, Isochinolyl, Imidazolyl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
- R(2): -(CH₂)ᵤ-(CF₂)ₜ-CF₃;
t Null, 1, 2 oder 3;
u Null oder 1;
- R(3): Wasserstoff oder unabhängig wie R(1) definiert;
sowie deren pharmazeutisch verträgliche Salze.

Bevorzugt sind Verbindungen der Formel I, in denen bedeuten:
- R(1): R(4)-SOₘ oder R(5)R(6)N-SO₂-;
m 1 oder 2;
R(4) und R(5)
   unabhängig voneinander Alkyl mit 1, 2, 3, 4, 5, 6, 7 oder 8 C-Atomen, CF₃ oder -CₙH₂ₙ-R(7);
   n Null, 1 oder 2;
R(6) H oder Alkyl mit 1, 2, 3 oder 4 C-Atomen;
R(7) Cycloalkyl mit 3, 4, 5, 6, 7 oder 8 C-Atomen;
oder
- R(1): -SR(10);
R(10)
Alkyl mit 1, 2, 3, 4, 5, 6, 7 oder 8 C-Atomen, -CₛH₂ₛ-(C₃-C₈)-Cycloalkyl oder ein aromatisches System ausgewählt aus der Gruppe bestehend aus Pyridyl, Pyrrolyl, Chinolyl, Isochinolyl, Imidazolyl oder Phenyl;
s Null, 1 oder 2; wobei die aromatischen Systeme Pyridyl, Pyrrolyl, Chinolyl, Isochinolyl, Imidazolyl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
- R(2): -(CF₂)ₜ-CF₃;
t Null oder 1;
- R(3): Wasserstoff oder unabhängig wie R(1) definiert;
sowie deren pharmazeutisch verträgliche Salze.

Ganz besonders bevorzugt sind Verbindungen der Formel I, in denen bedeuten:
- R(1): R(4)-SO₂ oder R(5)R(6)N-SO₂-;
R(4) und R(5)
unabhängig voneinander Alkyl mit 1, 2, 3, 4, 5, 6, 7 oder 8 C-Atomen, CF₃ oder Cycloalkyl mit 3, 4, 5, 6, 7 oder 8 C-Atomen;
R(6) H oder Alkyl mit 1, 2, 3 oder 4 C-Atomen;
oder
- R(1): -SR(10);
R(10)
Alkyl mit 1, 2, 3, 4, 5, 6, 7 oder 8 C-Atomen, -CₛH₂ₛ-(C₃-C₈)-Cycloalkyl oder ein aromatisches System ausgewählt aus der Gruppe bestehend aus Pyridyl, Pyrrolyl, Chinolyl, Isochinolyl, Imidazolyl oder Phenyl;
s Null, 1 oder 2; wobei die aromatischen Systeme Pyridyl, Pyrrolyl, Chinolyl, Isochinolyl, Imidazolyl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
- R(2): CF₃;
- R(3): Wasserstoff;
sowie deren pharmazeutisch verträgliche Salze.

Ganz speziell bevorzugt sind Verbindungen der Formel I, in denen bedeuten:
- R(1): R(4)-SO₂;
R(4) Alkyl mit 1, 2, 3 oder 4 C-Atomen oder Cycloalkyl mit 5 oder 6 C-Atomen;
oder
- R(1): -SR(10) oder -OR(10);
R(10)
Alkyl mit 1, 2, 3 oder 4 C-Atomen, (C₅-C₆)-Cycloalkyl oder ein aromatisches System ausgewählt aus der Gruppe bestehend aus Pyridyl, Pyrrolyl, Chinolyl, Isochinolyl, Imidazolyl oder Phenyl,
wobei die aromatischen Systeme Pyridyl, Pyrrolyl, Chinolyl, Isochinolyl, Imidazolyl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
- R(2): CF₃;
- R(3): Wasserstoff;
sowie deren pharmazeutisch verträgliche Salze.

Enthält einer der Substituenten R(1) bis R(12) ein oder mehrere Asymmetriezentren, so können diese sowohl S als auch R konfiguriert sein. Die Verbindungen können als optische Isomere, als Diastereomere, als Racemate oder als Gemische derselben vorliegen. Die bezeichneten Alkylreste können geradkettig oder verzweigt vorliegen.

Die Erfindung betrifft weiterhin ein Verfahren zur Herstellung der Verbindung I, dadurch gekennzeichnet, daß man eine Verbindung der Formel II 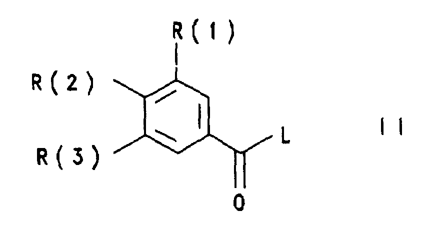 mit Guanidin umsetzt, worin R(1) bis R(3) die angegebene Bedeutung besitzen und L für eine leicht nucleophil substituierbare Fluchtgruppe steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäurederivaten (Formel II, L = OH) herstellen, wie beispielsweise die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit CI-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)-methylen)amino]-1,1,3,3-tetramethyluronium-tetrafluorborat ("TOTU") [Weiss und Krommer, Chemiker Zeitung 98, 817 (1974)]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Benzoesäuren werden nach einer der oben beschriebenen Verfahrensvarianten zu erfindungsgemäßen Verbindungen I umgesetzt.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine. Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 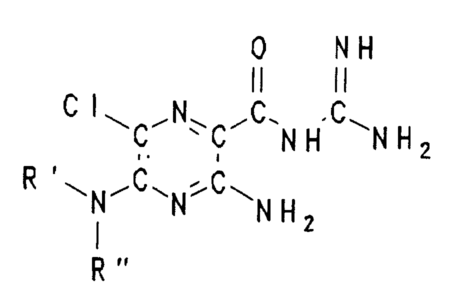
Amilorid: R', R'' = H
Dimethylamilorid: R',R'' = CH₃
Ethylisopropylamilorid: R'= C₂H₅, R'' = CH(CH₃)₂

Darüberhinaus sind Untersuchungen bekannt geworden, die auf antiarrhythmische Eigenschaften von Amilorid hinweisen (Circulation 79, 1257 - 63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 und in der US-Patentschrift 5 373 024 werden Benzoylguanidine beschrieben, die jedoch keine fluorierten Alkylsubstituenten aufweisen.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten, also auch keine fluorierten Verbindungen betreffen. Für diese Verbindungen wird eine starke salidiuretische Wirksamkeit berichtet.

Die deutsche Offenlegungsschrift P 43 05 250.9 beschreibt bereits Verbindungen, welche mit den erfindungsgemäßen verwandt sind, die jedoch in mancher Hinsicht noch nicht befriedigende Eigenschaften aufweisen. insbesondere sind darin noch keine Verbindungen mit fluorhaltigen Substituenten in p-Stellung beschrieben.

Die erfindungsgemäßen Verbindungen zeigen keine unerwünschten und nachteiligen salidiuretischen, jedoch sehr gute antiarrhythmische Eigenschaften aufweisen, wie sie beispielsweise für die Behandlung von Krankheiten wichtig sind, welche bei Sauerstoffmangelerscheinungen auftreten. Die Verbindungen I sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- RT: Raumtemperatur
- EE: Ethylacetat (EtOAc)
- Smp: Schmelzpunkt
- THF: Tetrahydrofuran
- eq.: Äquivalent

### Experimenteller Teil

Allgemeine Vorschrift zur Herstellung von Benzoyl-guanidinen (I)
Variante A: aus Benzoesäuren (II, L = OH)

1.0 eq. des Benzoesäurederivates der Formel II löst bzw. suspendiert man in wasserfreiem THF ( 5 ml/mmol) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotavapor) ab, versetzt mit Wasser, stellt mit 2N HCL auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.
Allgemeine Vorschrift zur Herstellung von Benzoyl-guanidinen (I)
Variante B: aus Benzoesäure-alkylestern (II, L = O-Alkyl)

1.0 eq. des Benzoesäure-alkylesters der Formel II sowie 5.0 eq. Guanidin (freie Base) werden in Isopropanol gelöst oder in THF suspendiert und bis zum vollständigen Umsatz (Dünnschichtkontrolle) zum Rückfluß erhitzt (typische Reaktionszeit 2 bis 5 h). Das Lösungsmittel wird unter vermindertem Druck (Rotavapor) abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.

### (Salzbildung vergleiche Variante A)

### Beispiel 1: 3-Methylsulfonyl-4-trifluormethyl-benzoylguanidin-hydrochlorid:

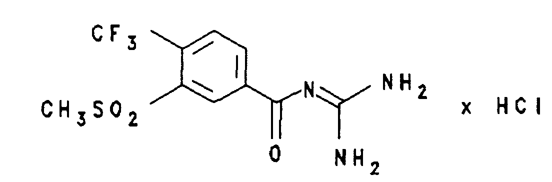

Farblose Kristalle, Smp. 236°C

### Syntheseweg:

a) 3-Methylsulfonyl-4-trifluormethyl-benzoesäuremethylester aus 4-Brom-3-methylsulfonyl-benzoesäuremethylester durch Erhitzen auf 90°C mit Kaliumtrifluoracetat in NMP in Gegenwart von Kupfer(I)iodid.
b) 3-Methylsulfonyl-4-trifluormethyl-benzoylguanidin-hydrochlorid nach allgemeiner Vorschrift, Variante B

## Patentansprüche

1. Benzoylguanidine der Formel I 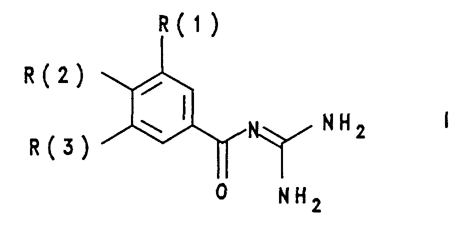 worin bedeuten:
R(1) R(4)-SOₘ oder R(5)R(6)N-SO₂-;
m 1 oder 2;
R(4) und R(5)
unabhängig voneinander Alkyl mit 1, 2, 3, 4, 5, 6, 7 oder 8 C-Atomen, Alkenyl mit 3, 4, 5 oder 6 C-Atomen, CF₃ oder -CₙH₂ₙ-R(7);
n Null, 1, 2, 3 oder 4;
R(6) H oder Alkyl mit 1, 2, 3 oder 4 C-Atomen;
R(7) Cycloalkyl mit 3, 4, 5, 6, 7 oder 8 C-Atomen oder Phenyl,
welches nicht substituiert ist oder substituiert mit 1 - 3 Substituenten ausgewählt aus der Gruppe bestehend aus F, Cl, CF₃, Methyl, Methoxy und NR(8)R(9);
R(8) und R(9)
H oder Alkyl mit 1, 2, 3 oder 4 C-Atomen;
oder
R(5) auch Wasserstoff;
oder
R(5) und R(6)
gemeinsam 4 oder 5 Methylengruppen, von denen eine CH₂-Gruppe durch Sauerstoff, S, NH, N-CH₃ oder N-Benzyl ersetzt sein kann;
oder
R(1) -SR(10);
R(10)
Wasserstoff, Alkyl mit 1, 2, 3, 4, 5, 6, 7 oder 8 C-Atomen,-CₛH₂ₛ-(C₃-C₈)-Cycloalkyl oder ein aromatisches System ausgewählt aus der Gruppe bestehend aus Pyridyl, Pyrrolyl, Chinolyl, Isochinolyl, Imidazolyl oder Phenyl;
s Null, 1 oder 2;
wobei die aromatischen Systeme Pyridyl, Pyrrolyl, Chinolyl, Isochinolyl, Imidazolyl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
R(2) -(CH₂)ᵤ-(CF₂)ₜ-CF₃;
t Null, 1, 2 oder 3;
u Null oder 1;
R(3) Wasserstoff oder unabhängig wie R(1) definiert;
sowie deren pharmazeutisch verträgliche Salze.

2. Verbindung der Formel I nach Anspruch 1, **dadurch gekennzeichnet, daß** darin bedeuten:
R(1) R(4)-SOₘ oder R(5)R(6)N-SO₂-;
m 1 oder 2;
R(4) und R(5)
unabhängig voneinander Alkyl mit 1, 2, 3, 4, 5, 6, 7 oder 8 C-Atomen, CF₃ oder -CₙH₂ₙ-R(7);
n Null, 1 oder 2;
R(6) H oder Alkyl mit 1, 2, 3 oder 4 C-Atomen;
R(7) Cycloalkyl mit 3, 4, 5, 6, 7 oder 8 C-Atomen;
oder
R(1) -SR(10);
R(10)
Alkyl mit 1, 2, 3, 4, 5, 6, 7 oder 8 C-Atomen, CₛH₂ₛ-(C₃-C₈)-Cycloalkyl oder ein aromatisches System ausgewählt aus der Gruppe bestehend aus Pyridyl, Pyrrolyl, Chinolyl, Isochinolyl, Imidazolyl oder Phenyl;
s Null, 1 oder 2;
wobei die aromatischen Systeme Pyridyl, Pyrrolyl, Chinolyl, Isochinolyl, Imidazolyl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
R(2) -(CF₂)ₜ-CF₃;
t Null oder 1;
R(3) Wasserstoff oder unabhängig wie R(1) definiert.

3. Verbindung der Formel I nach einem oder mehreren der Ansprüche 1 bis 2, **dadurch gekennzeichnet, daß** darin bedeuten:
R(1) R(4)-SO₂ oder R(5)R(6)N-SO₂-;
R(4) und R(5)
unabhängig voneinander Alkyl mit 1, 2, 3, 4, 5, 6, 7 oder 8 C-Atomen, CF₃ oder Cycloalkyl mit 3, 4, 5, 6, 7 oder 8 C-Atomen;
R(6) H oder Alkyl mit 1, 2, 3 oder 4 C-Atomen;
oder
R(1) -SR(10);
R(10)
Alkyl mit 1, 2, 3, 4, 5, 6, 7 oder 8 C-Atomen, -CₛH₂ₛ-(C₃-C₈)-Cycloalkyl oder ein aromatisches System ausgewählt aus der Gruppe bestehend aus Pyridyl, Pyrrolyl, Chinolyl, Isochinolyl, Imidazolyl oder Phenyl;
s Null, 1 oder 2;
wobei die aromatischen Systeme Pyridyl, Pyrrolyl, Chinolyl, Isochinolyl, Imidazolyl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
R(2) CF₃;
R(3) Wasserstoff.

4. Verbindung der Formel I nach einem oder mehreren der Ansprüche 1 bis 3, **dadurch gekennzeichnet, daß** darin bedeuten:
R(1) R(4)-SO₂;
R(4) Alkyl mit 1, 2, 3 oder 4 C-Atomen oder Cycloalkyl mit 5 oder 6 C-Atomen;
oder
R(1) -SR(10);
R(10)
Alkyl mit 1, 2, 3 oder 4 C-Atomen, (C₅-C₆)-Cycloalkyl oder ein aromatisches System ausgewählt aus der Gruppe bestehend aus Pyridyl, Pyrrolyl, Chinolyl, Isochinolyl, Imidazolyl oder Phenyl, wobei die aromatischen Systeme Pyridyl, Pyrrolyl, Chinolyl, Isochinolyl, Imidazolyl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
R(2) CF₃;
R(3) Wasserstoff.

5. Verfahren zur Herstellung einer Verbindung der Formel I nach Anspruch 1, **dadurch gekennzeichnet, daß** man
eine Verbindung der Formel II 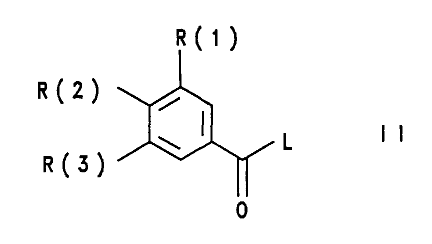 worin R(1) bis R(3) die in Anspruch 1 angegebene Bedeutung besitzen und L für eine leicht nucleophil substituierbare Fluchtgruppe steht, mit Guanidin umsetzt.

6. Verwendung einer Verbindung l nach Anspruch 1 zur Herstellung eines Medikaments zur Behandlung von Arrhythmien.

7. Verwendung einer Verbindung l nach Anspruch 1 zur Herstellung eines Medikaments zur Behandlung oder Prophylaxe des Herzinfarkts.

8. Verwendung einer Verbindung I nach Anspruch 1 zur Herstellung eines Medikaments zur Behandlung oder Prophylaxe der Angina Pectoris.

9. Verwendung einer Verbindung I nach Anspruch 1 zur Herstellung eines Medikaments zur Behandlung oder Prophylaxe von ischämischen Zuständen des Herzens.

10. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

11. Verwendung einer Verbindung I nach Anspruch 1 zur Herstellung eines Medikaments zur Behandlung oder Prophylaxe von ischämischen Zuständen peripherer Organe und Gliedmaßen.

12. Verwendung einer Verbindung I nach Anspruch 1 zur Herstellung eines Medikaments zur Behandlung von Schockzuständen.

13. Verwendung einer Verbindung I nach Anspruch 1 zur Herstellung eines Medikaments zum Einsatz bei chirurgischen Operationen und Organtransplantationen.

14. Verwendung einer Verbindung I nach Anspruch 1 zur Herstellung eines Medikaments zur Konservierung und Lagerung von Transplantaten für chirurgische Maßnahmen.

15. Verwendung einer Verbindung I nach Anspruch 1 zur Herstellung eines Medikaments zur Behandlung von Krankheiten, bei denen die Zellproliferation eine primäre oder sekundäre Ursache darstellt.

16. Heilmittel, enthaltend eine wirksame Menge einer Verbindung I nach einem oder mehreren der Ansprüche 1 bis 4.

## Claims

1. A benzoylguanidine of the formula I 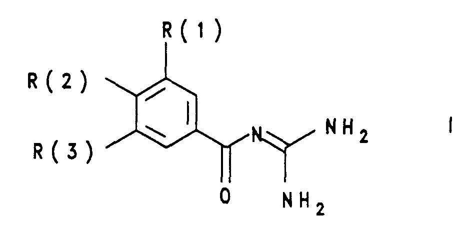 in which:
R(1) is R(4)-SOₘ or R(5)R(6)N-SO₂-;
m is 1 or 2;
R(4) and R(5)
independently of one another are alkyl having 1, 2, 3, 4, 5, 6, 7 or 8 carbon atoms, alkenyl having 3, 4, 5 or 6 carbon atoms, CF₃ or -CₙH₂ₙ-R(7);
n is zero, 1, 2, 3 or 4;
R(6) is H or alkyl having 1, 2, 3 or 4 carbon atoms;
R(7) is cycloalkyl having 3, 4, 5, 6, 7 or 8 carbon atoms, or phenyl which is unsubstituted or substituted by 1-3 substituents selected from the group consisting of F, Cl, CF₃, methyl, methoxy and NR(8)R(9);
R(8) and R(9)
are H or alkyl having 1, 2, 3 or 4 carbon atoms; or
R(5) is also hydrogen;
or
R(5) and R(6)
together are 4 or 5 methylene groups, of which a CH₂ group can be replaced by oxygen, S, NH, N-CH₃ or N-benzyl;
or
R(1) is -SR(10);
R(10)
is hydrogen, alkyl having 1, 2, 3, 4, 5, 6, 7 or 8 carbon atoms, -CₛH₂ₛ-(C₃-C₈)-cycloalkyl or an aromatic system selected from the group consisting of pyridyl, pyrrolyl, quinolyl, isoquinolyl, imidazolyl or phenyl;
s is zero, 1 or 2;
where the aromatic systems pyridyl, pyrrolyl, quinolyl, isoquinolyl, imidazolyl and phenyl are unsubstituted or substituted by 1-3 substituents selected from the group consisting of F, Cl, CF₃, CH₃, methoxy, hydroxyl, amino, methylamino and dimethylamino;
R(2) is -(CH₂)ᵤ-(CF₂)ₜ-CF₃;
t is zero, 1, 2 or 3;
u is zero or 1;
R(3) is hydrogen or independently defined as R(1);
or its pharmaceutically tolerable salts.

2. A compound of the formula I as claimed in claim 1, wherein:
R(1) is R(4)-SOₘ or R(5)R(6)N-SO₂-;
m is 1 or 2;
R(4) and R(5)
independently of one another are alkyl having 1, 2, 3, 4, 5, 6, 7 or 8 carbon atoms, CF₃ or -CₙH₂ₙ-R(7);
n is zero, 1 or 2;
R(6) is H or alkyl having 1, 2, 3 or 4 carbon atoms;
R(7) is cycloalkyl having 3, 4, 5, 6, 7 or 8 carbon atoms;
or
R(1) is -SR(10);
R(10)
is alkyl having 1, 2, 3, 4, 5, 6, 7 or 8 carbon atoms, -CₛH₂ₛ-(C₃-C₈)-cycloalkyl or an aromatic system selected from the group consisting of pyridyl, pyrrolyl, quinolyl, isoquinolyl, imidazolyl or phenyl;
s is zero, 1 or 2;
where the aromatic systems pyridyl, pyrrolyl, quinolyl, isoquinolyl, imidazolyl and phenyl are unsubstituted or substituted by 1-3 substituents selected from the group consisting of F, Cl, CF₃, CH₃, methoxy, hydroxyl, amino, methylamino and dimethylamino;
R(2) is -(CF₂)ₜ-CF₃;
t is zero or 1;
R(3) is hydrogen or independently defined as R(1).

3. A compound of the formula I as claimed in one or more of claims 1 and 2, wherein:
R(1) is R(4)-SO₂ or R(5)R(6)N-SO₂-;
R(4) and R(5)
independently of one another are alkyl having 1, 2, 3, 4, 5, 6, 7 or 8 carbon atoms, CF₃ or cycloalkyl having 3, 4, 5, 6, 7 or 8 carbon atoms;
R(6) is H or alkyl having 1, 2, 3 or 4 carbon atoms;
or
R(1) is -SR(10);
R(10)
is alkyl having 1, 2, 3, 4, 5, 6, 7 or 8 carbon atoms, -CₛH₂ₛ-(C₃-C₈)-cycloalkyl or an aromatic system selected from the group consisting of pyridyl, pyrrolyl, quinolyl, isoquinolyl, imidazolyl or phenyl;
s is zero, 1 or 2;
where the aromatic systems pyridyl, pyrrolyl, quinolyl, isoquinolyl, imidazolyl and phenyl are unsubstituted or substituted by 1-3 substituents selected from the group consisting of F, Cl, CF₃, CH₃, methoxy, hydroxyl, amino, methylamino and dimethylamino;
R(2) is CF₃;
R(3) is hydrogen.

4. A compound of the formula I as claimed in one or more of claims 1 to 3, wherein:
R(1) is R(4)-SO₂;
R(4) is alkyl having 1, 2, 3 or 4 carbon atoms or cycloalkyl having 5 or 6 carbon atoms;
or
R(1) is -SR(10)
R(10)
is alkyl having 1, 2, 3 or 4 carbon atoms, (C₅-C₆)-cycloalkyl or an aromatic system selected from the group consisting of pyridyl, pyrrolyl, quinolyl, isoquinolyl, imidazolyl or phenyl,
where the aromatic systems pyridyl, pyrrolyl, quinolyl, isoquinolyl, imidazolyl and phenyl are unsubstituted or substituted by 1-3 substituents selected from the group consisting of F, Cl, CF₃, CH₃, methoxy, hydroxyl, amino, methylamino and dimethylamino;
R(2) is CF₃;
R(3) is hydrogen.

5. A process for the preparation of a compound of the formula I as claimed in claim 1, which comprises
reacting a compound of the formula II 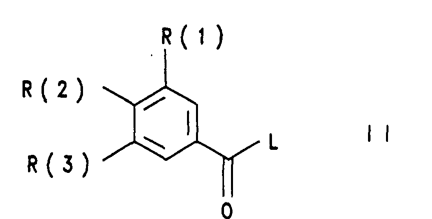 in which R(1) to R(3) have the meanings indicated in claim 1 and L is a leaving group which can be readily nucleophilically substituted, with guanidine.

6. The use of a compound I as claimed in claim 1 for the production of a medicament for the treatment of arrhythmias.

7. The use of a compound I as claimed in claim 1 for the production of a medicament for the treatment or prophylaxis of cardiac infarct.

8. The use of a compound I as claimed in claim 1 for the production of a medicament for the treatment or prophylaxis of angina pectoris.

9. The use of a compound I as claimed in claim 1 for the production of a medicament for the treatment or prophylaxis of ischemic conditions of the heart.

10. The use of a compound I as claimed in claim 1 for the production of a medicament for the treatment or prophylaxis of ischemic conditions of the peripheral and central nervous system and of stroke.

11. The use of a compound I as claimed in claim 1 for the production of a medicament for the treatment or prophylaxis of ischemic conditions of peripheral organs and limbs.

12. The use of a compound I as claimed in claim 1 for the production of a medicament for the treatment of states of shock.

13. The use of a compound I as claimed in claim 1 for the production of a medicament for use in surgical operations and organ transplantations.

14. The use of a compound I as claimed in claim 1 for the production of a medicament for the preservation and storage of transplants for surgical measures.

15. The use of a compound I as claimed in claim 1. for the production of a medicament for the treatment of illnesses in which cell proliferation is a primary or secondary cause.

16. A therapeutic comprising an efficacious amount of a compound I as claimed in one or more of claims 1 to 4.

## Revendications

1. Benzoylguanidines de formule I 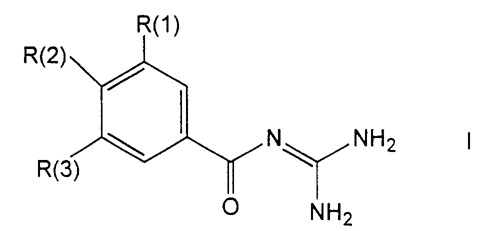 dans laquelle :
R(1) est R(4)-SOₘ ou R(5)R(6)N-SO₂- ;
m vaut 1 ou 2 ;
R(4) et R(5)
sont indépendamment l'un de l'autre un radical alkyle avec 1, 2, 3, 4, 5, 6, 7 ou 8 atomes de carbone, alcényle avec 3, 4, 5 ou 6 atomes de carbone, CF₃ ou -CₙH₂ₙ-R(7) ;
n vaut zéro, 1, 2, 3 ou 4 ;
R(6) est H ou un radical alkyle avec 1, 2, 3 ou 4 atomes de carbone ;
R(7) est un radical cycloalkyle avec 3, 4, 5, 6, 7 ou 8 atomes de carbone ou phényle, qui n'est pas substitué ou qui est substitué par 1 - 3 substituants choisis dans le groupe constitué par F, Cl, CF₃, un radical méthyle, méthoxy et NR(8)R(9) ;
R(8) et R(9) sont H ou un radical alkyle avec 1, 2, 3 ou 4 atomes de carbone ;
ou
R(5) est également l'hydrogène ;
ou
R(5) et R(6)
sont ensemble des radicaux 4 ou 5 méthylène, dont un radical CH₂- peut être remplacé par un oxygène, S, NH, N-CH₃ ou N-benzyle;
ou
R(1) est -SR(10) ;
R(10) est un hydrogène, un radical alkyle avec 1, 2, 3, 4, 5, 6, 7 ou 8 atomes de carbone, -CₛH₂ₛ-(C₃-C₈) -cycloalkyle ou un système aromatique choisi dans le groupe constitué par les radicaux pyridyle, pyrrolyle, quinolyle, isoquinolyle, imidazolyle ou phényle ;
s vaut zéro, 1 ou 2;
les systèmes aromatiques pyridyle, pyrrolyle, quinolyle, isoquinolyle, imidazolyle et phényle étant non substitués ou substitués par 1 - 3 substituants choisis dans le groupe constitué par F, Cl, les radicaux CF₃, CH₃, méthoxy, hydroxy, amino, méthylamino et diméthylamino;
R(2) est -(CH₂)ᵤ-(CF₂)ₜ-CF₃;
t vaut zéro, 1, 2 ou 3;
u vaut zéro ou 1;
R(3) est l'hydrogène ou est défini indépendamment comme R(1);
ainsi que leurs sels acceptables du point de vue pharmaceutique.

2. Composé de formule I selon la revendication 1, **caractérisé en ce que** :
R(1) est R(4)-SOₘ ou R(5)R(6)N-SO₂-;
m vaut 1 ou 2;
R(4) et R(5)
sont indépendamment l'un de l'autre un radical alkyle avec 1, 2, 3, 4, 5, 6, 7 ou 8 atomes de carbone, CF₃ ou -CₙH₂ₙ-R(7) ;
n vaut zéro, 1, ou 2;
R(6) est H ou un radical alkyle avec 1, 2, 3 ou 4 atomes de carbone ;
R(7) est un radical cycloalkyle avec 3, 4, 5, 6, 7 ou 8 atomes de carbone ;
ou
R(1) est -SR(10);
R(10) est un radical alkyle avec 1, 2, 3, 4, 5, 6, 7 ou 8 atomes de carbone,
-CₛH₂ₛ-(C₃-C₈)-cycloalkyle ou un système aromatique choisi dans le groupe constitué par les radicaux pyridyle, pyrrolyle, quinolyle, isoquinolyle, imidazolyle ou phényle ;
s vaut zéro, 1 ou 2;
les systèmes aromatiques pyridyle, pyrrolyle, quinolyle, isoquinolyle, imidazolyle et phényle étant non substitués ou substitués par 1 - 3 substituants choisis dans le groupe constitué par F, Cl, les radicaux CF₃, CH₃, méthoxy, hydroxy, amino, méthylamino et diméthylamino;
R(2) est -(CF₂)ₜ-CF₃;
t vaut zéro ou 1;
R(3) est l'hydrogène ou est défini indépendamment comme R(1).

3. Composé de formule I selon l'une ou plusieurs des revendications 1 à 2, **caractérisé en ce que** :
R(1) est R(4)-SO₂ ou R(5)R(6)N-SO₂- ;
R(4) et R(5)
sont indépendamment l'un de l'autre un radical alkyle avec 1, 2, 3, 4, 5, 6, 7 ou 8 atomes de carbone, CF₃ ou un radical cycloalkyle avec 3, 4, 5, 6, 7 ou 8 atomes de carbone ;
R(6) est H ou un radical alkyle avec 1, 2, 3 ou 4 atomes de carbone ;
ou
R(1) est -SR(10);
R(10) est un radical alkyle avec 1, 2, 3, 4, 5, 6, 7 ou 8 atomes de carbone, -CₛH₂ₛ-(C₃-C₈)-cycloalkyle ou un système aromatique choisi dans le groupe constitué par les radicaux pyridyle, pyrrolyle, quinolyle, isoquinolyle, imidazolyle ou phényle ;
s vaut zéro, 1 ou 2;
les systèmes aromatiques pyridyle, pyrrolyle, quinolyle, isoquinolyle, imidazolyle et phényle étant non substitués ou substitués par 1 - 3 substituants choisis dans le groupe constitué par F, Cl, les radicaux CF₃, CH₃, méthoxy, hydroxy, amino, méthylamino et diméthylamino;
R(2) est CF₃;
R(3) est l'hydrogène.

4. Composé de formule I selon l'une ou plusieurs des revendications 1 à 3, **caractérisé en ce que** :
R(1) est R(4)-SO₂;
R(4) est un radical alkyle avec 1, 2, 3 ou 4 atomes de carbone ou cycloalkyle avec 5 ou 6 atomes de carbone ;
ou
R(1) est-SR(10);
R(10) est un radical alkyle avec 1, 2, 3 ou 4 atomes de carbone, (C₅-C₆)-cycloalkyle ou un système aromatique choisi dans le groupe constitué par les radicaux pyridyle, pyrrolyle, quinolyle, isoquinolyle, imidazolyle ou phényle ;
où les systèmes aromatiques pyridyle, pyrrolyle, quinolyle, isoquinolyle, imidazolyle et phényle étant non substitués ou substitués par 1 - 3 substituants choisis dans le groupe constitué par F, Cl, les radicaux CF₃, CH₃, méthoxy, hydroxy, amino, méthylamino et diméthylamino;
R(2) est CF₃;
R(3) est l'hydrogène.

5. Procédé de préparation d'un composé de formule I selon la revendication 1, **caractérisé en ce qu'**on fait réagir un composé de formule II 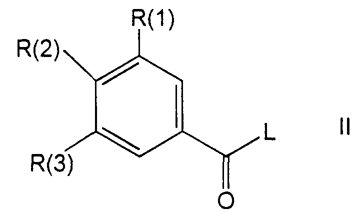 avec la guanidine, formule dans laquelle R(1) à R(3) ont la signification donnée et L est un groupe partant qui peut être substitué facilement de manière nucléophile.

6. Utilisation d'un composé de formule I tel que revendiqué dans la revendication 1 pour la préparation d'un médicament pour le traitement des arythmies.

7. Utilisation d'un composé de formule I tel que revendiqué dans la revendication 1 pour la préparation d'un médicament pour le traitement ou la prophylaxie de l'infarctus du myocarde.

8. Utilisation d'un composé de formule l tel que revendiqué dans la revendication 1 pour la préparation d'un médicament pour le traitement ou la prophylaxie de l'angine de poitrine.

9. Utilisation d'un composé de formule I tel que revendiqué dans la revendication 1 pour la préparation d'un médicament pour le traitement ou la prophylaxie des états ischémiques du coeur.

10. Utilisation d'un composé de formule I tel que revendiqué dans la revendication 1 pour la préparation d'un médicament pour le traitement ou la prophylaxie des états ischémiques du système nerveux périphérique et central et de l'apoplexie.

11. Utilisation d'un composé de formule I tel que revendiqué dans la revendication 1 pour la préparation d'un médicament pour le traitement ou la prophylaxie des états ischémiques des organes périphériques et des extrémités.

12. Utilisation d'un composé de formule I tel que revendiqué dans la revendication 1 pour la préparation d'un médicament pour le traitement d'états de chocs.

13. Utilisation d'un composé de formule I tel que revendiqué dans la revendication 1 pour la préparation d'un médicament pour l'emploi dans des opérations chirurgicales et des transplantations d'organes.

14. Utilisation d'un composé de formule I tel que revendiqué dans la revendication 1 pour la préparation d'un médicament pour la conservation et le stockage d'organes à transplanter pour des opérations chirurgicales.

15. Utilisation d'un composé de formule I tel que revendiqué dans la revendication 1 pour la préparation d'un médicament pour le traitement de maladies pour lesquelles une prolifération cellulaire constitue une cause primaire ou secondaire.

16. Médicament comprenant une quantité efficace d'un composé de formule I tel que revendiqué dans l'une ou plusieurs des revendications 1 à 4.
